# EUROPEAN PATENT APPLICATION

(11) **EP 4 751 767 A1**
(43) Date of publication of application: **03.06.2026**
(21) Application number: 24216785.6
(22) Date of filing: 02.12.2024
(51) Int. Cl.: A61N 1/39, A61B 5/00, A61H 31/00, A61B 5/0205

(54) **ENHANCED RESUSCITATION FOR EMERGENCY CARE**

(71) Applicant: Koninklijke Philips N.V., 5656 AE Eindhoven (NL)
(72) Inventor: BERMAN, Randall M, 5656 AG Eindhoven (NL); FUJITO, Keiichi, 5656 AG Eindhoven (NL); ARBANAS, Brian, 5656 AG Eindhoven (NL); JORGENSON, Dawn Blilie, 5656 AG Eindhoven (NL); FROSH, Karl, 5656 AG Eindhoven (NL); GEHMAN, Stacy Earl, 5656 AG Eindhoven (NL); SZYMANSKI, Joyce Ann, 5656 AG Eindhoven (NL); BURNS, Delaney, 5656 AG Eindhoven (NL); EVANS, Dave, 5656 AG Eindhoven (NL); RIEMER, Adena, 5656 AG Eindhoven (NL); DE LAT, Bente, 5656 AG Eindhoven (NL)
(74) Representative: Philips Intellectual Property & Standards

(57) **Abstract**

A defibrillation system employs one or more of a wearable biosensor (40) configured to monitor a ventilation of the patient, a wearable injector (50) configured to administer a medication to the patient, a digital stethoscope (60) configured to monitor a heart of the patient; and ambient sensor (70, 80)(s) configured to monitor ambient sound and/or ambient light. The defibrillation system further employs a defibrillator (20) including a defibrillation controller (21) configured to implement a resuscitation protocol in accordance with one or more of a monitoring of the ventilation of the patient by the wearable biosensor (40), an administering of the medication to the patient by the wearable injector (50), a monitoring of the heart of the patient by the digital stethoscope (60), and a monitoring of one of ambient sound and ambient light by the at least one ambient sensor (70, 80).

## Description

### FIELD OF THE INVENTION

The present disclosure generally relates to a resuscitation of a patient. The present disclosure particularly relates to an enhancement to a defibrillator execution of a resuscitation protocol.

### BACKGROUND OF THE INVENTION

Cardiopulmonary resuscitation (CPR) is a lifesaving intervention that is provided when a patient is in cardiac arrest (e.g., with no or ineffective mechanical activity of the patient's heart) and primarily consists of chest compressions often combined with ventilation. A defibrillator may be used during CPR to deliver a high-amplitude current impulse to the heart in order to restore normal rhythm and contractile function in a patient experiencing an arrhythmia (e.g., ventricular fibrillation (VF) and ventricular tachycardia (VT)) that is not accompanied by a palpable pulse. There are several classes of defibrillators that are particularly useful during CPR of a patient including a monitor/defibrillator, an automatic external defibrillator, a semi-automatic external defibrillator, a manual external defibrillator, and an advanced life support defibrillator. The CPR/defibrillation industry is constantly striving to improve upon CPR/defibrillation technology, particularly as related to the enhancement of CPR protocols and shock protocols.

### SUMMARY OF THE INVENTION

The present disclosure is directed to an analysis and annotations by a defibrillator of resuscitation events from patients suffering sudden cardiac arrest (SCA) and receiving cardiopulmonary resuscitation. Examples of resuscitation events in accordance with the present disclosure include a diagnostic defibrillation event, a diagnostic ventilation event and a diagnostic CPR event as set forth in the present disclosure.

The present disclosure may be embodied as (1) a defibrillation system, (2) a defibrillation controller and (2) a defibrillation method.

Various exemplary embodiments of a defibrillation system of the present disclosure employ A defibrillation system employs one or more of (1) a wearable biosensor configured to monitor a ventilation of the patient, (2) a wearable injector configured to administer a medication to the patient, (3) a digital stethoscope configured to monitor a heart of the patient; and (4) ambient sensor(s) configured to monitor ambient sound and/or ambient light.

The various embodiments of a defibrillation system in accordance with the present disclosure further employ a defibrillator including a defibrillation controller configured to implement a resuscitation protocol in accordance with one or more of (1) a monitoring of the ventilation of the patient by the wearable biosensor, (2) an administering of the medication to the patient by the wearable injector, (3) a monitoring of the heart of the patient by the digital stethoscope, and (4) a monitoring of the ambient sound and/or the ambient light by the ambient sensor(s).

Various exemplary embodiments of a defibrillation controller of the present disclosure employ a non-transitory machine-readable storage medium encoded with instructions executable by one or more processors for controlling an implementation by a defibrillator of a resuscitation protocol including a cardiopulmonary resuscitation protocol (CPR) and a shock protocol.

The non-transitory machine-readable storage medium includes the instructions to establish communication with one or more of (1) a wearable biosensor configured to monitor a ventilation of the patient, (2) a wearable injector configured to administer a medication to the patient, (3) a digital stethoscope configured to monitor a heart of the patient, and (4) ambient sensor(s) configured to monitor ambient sound and/or ambient light.

The non-transitory machine-readable storage medium further includes the instructions to control the implementation of the resuscitation protocol by the defibrillator in accordance with one or more of (1) a monitoring of the ventilation of the patient by the wearable biosensor, (2) an administering of the medication to the patient by the wearable injector, (3) a monitoring of the heart of the patient by the digital stethoscope, and (4) a monitoring of the ambient sound and/or the ambient light by ambient sensor(s).

Various exemplary embodiments of a defibrillation method of the present disclosure involves a defibrillator implementing a resuscitation protocol in accordance with one or more of (1) a monitoring of the ventilation of the patient by the wearable biosensor, (2) an administering of the medication to the patient by the wearable injector, (3) a monitoring of the heart of the patient by the digital stethoscope, and (4) a monitoring of an ambient sound and an ambient light by ambient sensor(s).

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates an exemplary embodiment of a defibrillation system in accordance with the present disclosure;
FIG. 2 illustrates an exemplary embodiment of a defibrillation controller in accordance with the present disclosure;
FIG. 3 illustrates a flowchart representative of an exemplary embodiment of a resuscitation protocol in accordance with the present disclosure;
FIG. 4 illustrates a flowchart representative of an exemplary embodiment of an instruction language management in accordance with the present disclosure; and
FIG. 5 illustrates a flowchart representative of an exemplary embodiment of an agonal gasping therapy in accordance with the present disclosure.

### DETAILED DESCRIPTION OF THE PREFERRED EMBODIMENTS

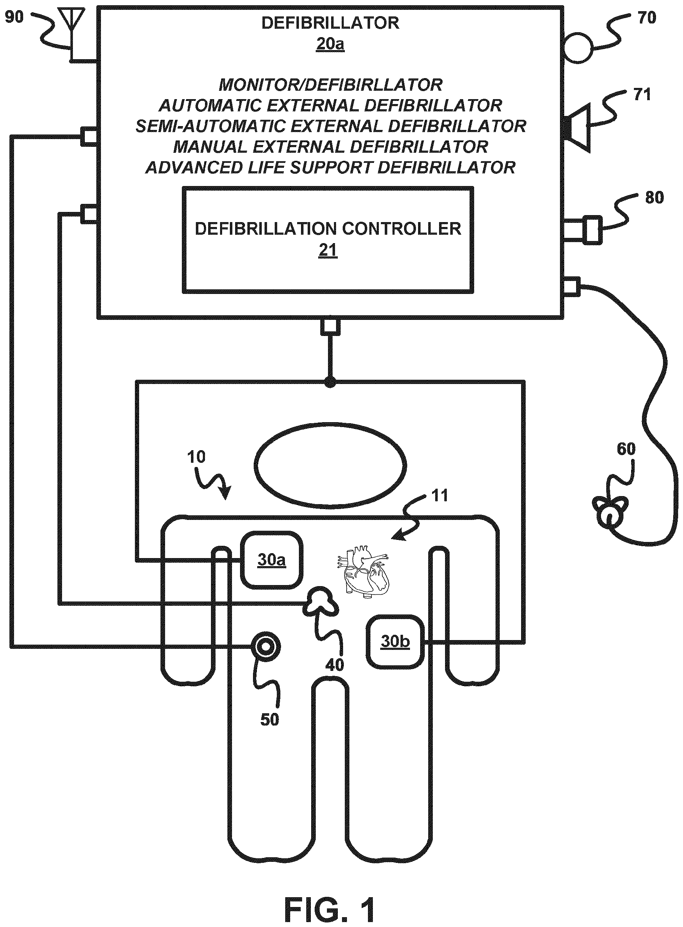

To facilitate an understanding of the present disclosure, the following description of FIG. 1-3 teaches exemplary embodiments of devices, systems and methods in accordance with the present disclosure. From the following description of FIGS. 1-3, those having ordinary skill in the art of the present disclosure will appreciate how to apply the present disclosure to make and use additional embodiments of devices, systems and methods in accordance with the present disclosure.

FIG. 1 illustrates an exemplary defibrillator system of the present disclosure employs a defibrillator 20, a pair of electrode pads 30a and 30b, a wearable biosensor 40, a wearable injector 50, a digital stethoscope 60, a microphone 70 and a light sensor 80. The following description of the exemplary defibrillator system will be in the context of a cardiopulmonary resuscitation (CPR) of a heart 11 of a patient 10 as shown in FIG. 1. Nonetheless, in practice, defibrillation systems of the present disclosure can be utilized for emergency care and other medical events requiring a defibrillator.

Referring to FIG. 1, for purposes of describing and claiming the present disclosure, defibrillator 20 broadly encompasses any defibrillator, as known in the art of the present disclosure and hereinafter conceived, configured for executing a resuscitation protocol in accordance with the present disclosure. Non-limiting examples of defibrillator 20 include a defibrillator/monitor, an automatic external defibrillator, a semi-automatic external defibrillator, a manual external defibrillator and an advanced life support defibrillator.

Defibrillator 20 includes a defibrillation controller 21 for controlling the execution of a resuscitation protocol based on electrocardiogram (ECG) signals received from electrode pads 30a and 30b as known in the art of the present disclosure and further based on data and signals communicated to defibrillator 20 by wearable biosensor 40, wearable injector 50, digital stethoscope 60, microphone 70 and light sensor 80 in accordance with the present disclosure.

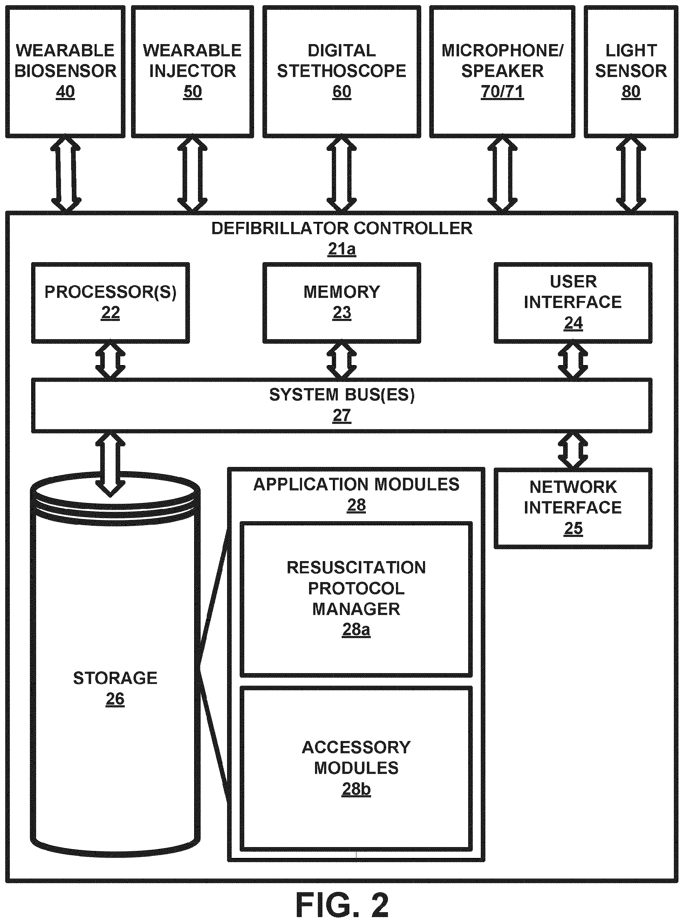

FIG. 2 illustrates an exemplary embodiment 20a of controller 21 (FIG. 1) that includes one or more processor(s) 22, memory 23, a user interface 24, a network interface 25, and a storage 26 interconnected via one or more system bus(es) 27.

Referring to FIG. 2, processor 22 can be any hardware device, as known in the art of the present disclosure or hereinafter conceived, capable of executing instructions stored in memory 23 or storage or otherwise processing data. In a non-limiting example, the processor(s) 22 can include a microprocessor, field programmable gate array (FPGA), application-specific integrated circuit (ASIC), or other similar devices.

The memory 23 can include various memories, as known in the art of the present disclosure or hereinafter conceived, including, but not limited to, L1, L2, or L3 cache or system memory. In a non-limiting example, the memory 23 can include static random access memory (SRAM), dynamic RAM (DRAM), flash memory, read only memory (ROM), or other similar memory devices.

The user interface 24 can include one or more devices, as known in the art of the present disclosure or hereinafter conceived, for enabling communication with a user such as an administrator. In a non-limiting example, the user interface can include a command line interface or graphical user interface that can be presented to a remote terminal via the network interface 25.

The network interface 25 can include one or more devices, as known in the art of the present disclosure or hereinafter conceived, for enabling communication other components of a medical device. In a non-limiting example, the network interface 25 can include a network interface card (NIC) configured to communicate according to the Ethernet protocol. Additionally, the network interface 25 may implement a TCP/IP stack for communication according to the TCP/IP protocols. Various alternative or additional hardware or configurations for the network interface 25 will be apparent.

The storage 26 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 26 can store instructions for execution by the processor(s) 22 or data upon with the processor(s) 22 may operate. For example, the storage 26 may store a base operating system for controlling various basic operations of the hardware.

The storage 26 can also store application modules 28 in the form of executable software/firmware for implementing the principles as previously described in the present disclosure.

In one exemplary embodiment as shown, storage 26 stores application modules 28 including resuscitation protocol manager 28a and accessory modules 28b for operatively communicating/interacting with wearable biosensor 40, wearable injector 50, digital stethoscope 60, microphone 70 and light sensor 80 as known in the art of the present disclosure and hereinafter conceived.

Referring back to FIG. 1, for purposes of describing and claiming the present disclosure, electrode pads 30a and 30b broadly encompass any electrode pads, as known in the art of the present disclosure and hereinafter conceived, utilized by defibrillator 20 for establishing conductive pathway in patient 10 to facilitate a monitoring of electrical activity of heart 11 of patient 10 and a delivery of a defibrillation shock to heart 11 of patient 10.

Referring back to FIG. 1, for purposes of describing and claiming the present disclosure, wearable biosensor 40 broadly encompass any wearable device, as known in the art of the present disclosure and hereinafter conceived, for monitoring ventilation of patient 10 during resuscitation and emergency/critical care.

In practice of the present disclosure, wearable biosensor 40 can be a stand-alone accessary of defibrillator 20 whereby wearable biosensor 40 can wired/wirelessly communicated with defibrillator 40, or wearable biosensor 40 can have a cable integrated into defibrillator 20.

Also in practice of the present disclosure, wearable biosensor 40 is operated to count ventilations applied to patient 10 during a resuscitation of patient 10, and defibrillation controller 21 utilizes the ventilation count or lack thereof to systematically adjust the resuscitation protocol if needed.

In one example, defibrillation controller 21 can utilize the ventilation count to derive a ventilation rate for determining if patient 10 is being over ventilated or under ventilated, whereby defibrillation controller 21 will provide a communication to the responder(s) decrease the rate of ventilation if the ventilation rate indicates patient10 is being over ventilated or to increase the rate of ventilation if the ventilation rate indicates patient 10 is being under ventilated. The over/under ventilated determination can be accomplished by comparing the ventilation rate to a ventilation profile associated with patient 10 (e.g., a generic profile or a customed profile based on gender and/or age), or by comparing the ventilation rate to over/under ventilated thresholds established from training data associated with ventilation of patients.

In another example, defibrillation controller 21 utilizes a lack of any ventilation count or a ceasing of the ventilation count as an indication of a patient 10 has stopped breathing or as an indication the ventilator (e.g., bag valve mask) is leaking/unsealed and/or improperly positioned/aligned within patient 10, whereby the defibrillation controller 21 will provide a communication to the responder(s) to check the breathing status of patient 10 followed by checking the operational conditions of the ventilator.

In yet another example, if wearable biosensor 40 is configured to implement an acoustic resonance technique as known in the art of the present disclosure or hereinafter conceived, then defibrillation controller 21 can utilize an acoustic resonance signal from wearable biosensor 40 to derive a ventilation volume of patient 10 for determining if patient 10 is having a difficulty breathing or shortness of breath due to a low ventilation volume or a high ventilation volume, whereby defibrillation controller 21 can communicate to the responder(s) to check the breathing status of patient 10. The low/high ventilation volume can be accomplished by comparing the ventilation volume to a ventilation profile associated with patient 10 (e.g., a generic profile or a customed profile based on gender and/or age), or by comparing the ventilation volume to low/high ventilation volume thresholds established from training data associated with ventilation of patients. Furthermore, defibrillation controller 21 can utilize the acoustic resonance signal to assess crackles, pneumonia, pneumothorax and other conditions whereby defibrillation controller 21 can communicate to the responder(s) to check the breathing status of patient 10.

In yet another example, defibrillation controller 21 can utilize the ventilation count and/or ventilation volume to track breathing changes of patient 10 during the course of the CPR and/or patient monitoring, particularly, any effect of medication(s) provided to patient 10 during the CPR) and/or patient monitoring.

Referring back to FIG. 1, for purposes of describing and claiming the present disclosure, wearable injector 50 broadly encompass any wearable device, as known in the art of the present disclosure and hereinafter conceived, for injecting medication(s) into patient 10 during the course of CPR being applied to patient 10.

In practice of the present disclosure, wearable injector 50 can be a stand-alone accessary of defibrillator 20 whereby wearable injector 50 can wired/wirelessly communicated with defibrillator 40, or wearable injector 50 can have a cable integrated into defibrillator 20.

Also in practice of the present disclosure, wearable injector 50 is operated by defibrillation controller 21 to, in addition to providing medication(s) to patient 10, record the timing and amount of the medications as well as record ventilations and observations and diagnostics made from the acoustic resonance signal.

Defibrillation controller 21 can utilize such medication information from wearable injector 50 to analyze a response by patient 10 to the medication (e.g., heart rate, respiratory rate, changes in ECG) and used as input to various techniques implemented within the resuscitation protocol (e.g., shock advisory techniques and/or shock delivery techniques).

In one example with patient 10 experiencing cardiac arrest, the administration of epinephrine and antiarrhythmics by wearable injector 50 as controlled defibrillation controller 21, and their effect could be used by defibrillation controller 30 to optimally guide the resuscitation protocol for specific treatment(s) of patient 10.

Further in practice, the medication information can be stored in electronic record(s) of patient 10 stored locally or remotely (via Bluetooth and/or the cloud), which can provide for improved QA/QI and tracking of the cardiac status of patient 10.

Still referring to FIG. 1, for purposes of describing and claiming the present disclosure, digital stethoscope 60 broadly encompass any stethoscope, as known in the art of the present disclosure and hereinafter conceived, for converting acoustic signals from heart 11, lungs and/or thorax of patient 10 into digital signals indicative of a cardiac status of heart 11 of patient 10.

In practice of the present disclosure, digital stethoscope 60 can be a stand-alone accessary of defibrillator 20 whereby digital stethoscope 60 can wired/wirelessly communicated with defibrillator 40, or digital stethoscope 60 can have a cable integrated into defibrillator 20.

Also in practice, defibrillation controller 21 can control a display of the digital signals from digital stethoscope 60 on a display screen of defibrillator 20 and can utilize such digital cardiac signals and lung sounds as input to various techniques implemented within the resuscitation protocol (e.g., shock advisory techniques, shock delivery techniques and/or return of spontaneous circulation techniques).

Further in practice, the digital cardiac signals can be stored in electronic record(s) of patient 10 stored locally or remotely (via Bluetooth and/or the cloud), which can provide for improved QA/QI and tracking of the cardiac status of patient 10.

Still referring to FIG. 1, for purposes of describing and claiming the present disclosure, microphone 70 and light sensor 80 broadly encompass any devices, as known in the art of the present disclosure and hereinafter conceived, for respectively detecting ambient sound and ambient light.

In practice, microphone 70 and light sensor 80 can be built in sensors of defibrillator 20, whereby ambient light and ambient sound can be continuously or intermittently detected to thereby facilitate a systematic adjustment to a sound communication by defibrillator 20 due to background noise (e.g., volume adjustments voice prompts, metronome, beeps, etc.) and to thereby further facilitate a systematic adjustment to visual communication by defibrillator 20 due to ambient lighting (e.g., brightness adjustments of ECG screen, buttons and pad icons, and font size/image size adjustments of ECG screen).

For example, if defibrillator 20 is being operated near a train station and a train goes by, then the prompts would automatically become louder as the train is passing and the volume would decrease after the train had passed.

By further example, if defibrillator 20 is being operated in a dark room or in bright sunshine, the lighting of the ECG screen, buttons and pad icons can be increased or decreased accordingly.

The sound/light adjustments can be accomplished by comparing the detected ambient sound and ambient light to a sound/lighting profile deemed suitable for an optimal operation of defibrillator 20, or by comparing the detected ambient sound and ambient light to established from training data associated with an operation of defibrillator 20.

Further, in accordance with the present disclosure, microphone 70 can be used to by defibrillator 20 to detect a language and/or dialect being spoken by a user of the device and/or any person within the acoustic range of microphone 70, and determine if the detected language and/or dialect is the same as the language and/or dialect set for audio outputs (including, e.g., instructions and process flow). If defibrillator 20 determines that the detected language and/or dialect is different than the language and/or dialect set for audio outputs, then defibrillator 20 would, via an audio output device/speaker of defibrillator 20, ask, in the detected language and/or dialect, if the user would like for the defibrator to provide audio outputs, such as instructions, in the detected language and/or dialect. Upon the user answering in the affirmative, in the detected language and/or dialect and/or any other language and/or dialect, then defibrator 20 can/would automatically switch to provide audio output in the detected language and/or dialect.

Further still, in addition to providing for switching to the detected language and/or dialect, defibrillator 20 can/would automatically adjust the instructions based on the culture associated with the detected language and/or dialect. For example, if the culture identified by defibrillator 20 based on the detected language and/or dialect is prone to never removing a person's clothing in public, the adjusted instructions can include additional prompting to the user to remove the clothing to expose the chest for placing the electrodes/pad, emphasizing that it is acceptable to do so in this emergency situation to help save the patient's life. These adjusted instructions can be organized, for example, in preconfigured profiles corresponding to different languages and/or dialects.

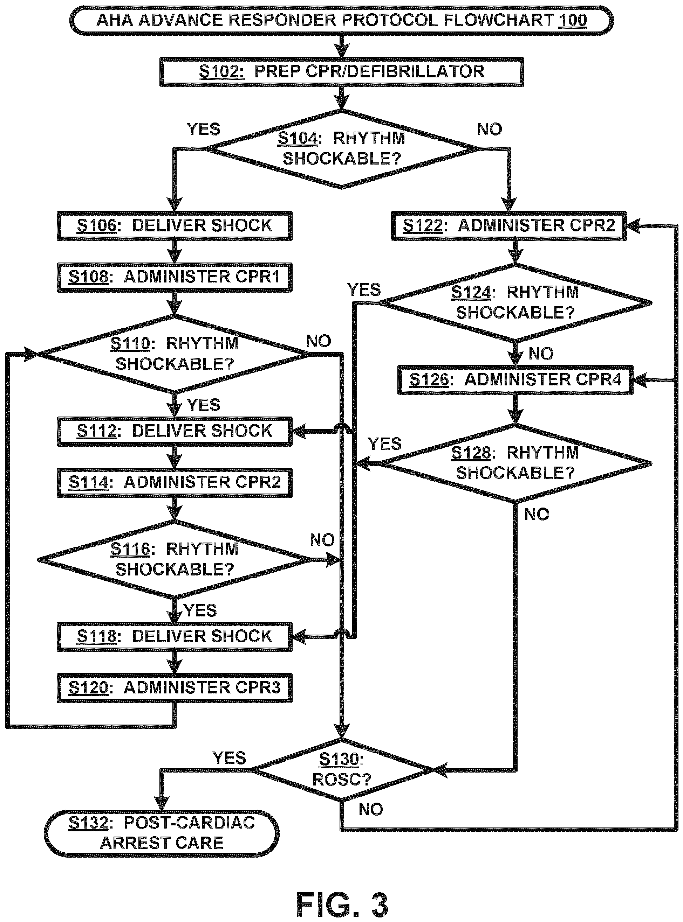

FIG. 3 depicts a flowchart 100 representative of an exemplary American Heart Association (AHA) advanced responder protocol (i.e., resuscitation protocol) as known in the art of the present disclosure.

Referring to FIG. 3, a stage S102 of flowchart 100 encompasses a preparation of an administration of CPR by the responder to a patient experiencing cardiac arrest. In one embodiment of stage S102, the responder can commence providing oxygen to the patient and attach an Advanced Life Support (ALS) monitor/defibrillator to the patient.

A stage S104 of flowchart 100 encompasses a determination of whether an initial cardiac rhythm of an electrocardiogram (ECG) of the patient is a shockable cardiac rhythm or a non-shockable cardiac rhythm. If the cardiac rhythm of the ECG of the patient is determined to be a shockable cardiac rhythm during stage S104, then a stage S106 of flowchart 100 encompasses a delivery of a first shock to the patient and a stage S108 of flowchart 100 encompasses an administration of a first CPR protocol to the patient.

In one embodiment of stage S106, a biphasic shock energy of 120-200 joules can be delivered to the patient, or alternatively, a maximum available biphasic shock energy. In a second embodiment of stage S106, a monophasic shock energy of 360 joules can be delivered to the patient.

In one embodiment of stage S108, the responder is guided to administer a quality CPR to the patient for a set time period (e.g., two (2) minutes). For example, during the set time period, the responder can be guided to push hard (e.g., 2-2.4 inches/5-6 centimeters) and fast (e.g., 100-120 compressions/minute) while allowing for a complete chest recoil. Furthermore, interruptions in compressions should be minimized, excessive ventilation should be avoided and a compressor, if applicable, should be rotated at the end of the set time period. Moreover, a 30:2 compression-ventilation ratio should be maintained if the patient does not have an advanced airway, a maximum partial pressure of CO₂ at the end a breath should be ≥ 10 mm Hg, and a relaxation phase diagnostic pressure should be ≥ 20 mm Hg. Additionally, IV/IO access to the patient can be established.

Still referring to FIG. 3, subsequent to an expiration of the set time period of stage S108, a stage S110 of flowchart 100 encompasses a determination of whether an ongoing cardiac rhythm of the ECG of the patient is a shockable cardiac rhythm or a non-shockable cardiac rhythm. If the ongoing cardiac rhythm of ECG of the patient is determined to be a shockable cardiac rhythm during stage S110, then a stage S 112 of flowchart 100 encompasses a delivery of a second shock to the patient and a stage S114 of flowchart 100 encompasses an administration of a second CPR protocol to the patient.

In one embodiment of stage S112, a biphasic shock energy of at least 120-200 joules can be delivered to the patient, or alternatively, a maximum available biphasic shock energy. In a second embodiment of stage S112, a monophasic shock energy of 360 joules can be delivered to the patient.

In one embodiment of stage S114, the responder is guided to administer a quality CPR to the patient as set forth in the first CPR protocol of stage S108 for a set time period (e.g., two (2) minutes). Additionally, a Epinephrine IV/IO dose of 1mg can be delivered every 3-5 minutes to the patient by the responder and advanced airway capnography can be considered (e.g., supraglottic advanced airway or endotracheal intubation, and/or waveform capnography to confirm and monitor ET tube placement, and/or 8-10 breaths per minute with continuous chest compressions).

Still referring to FIG. 3, subsequent to an expiration of the set time period of stage S114, a stage S116 of flowchart 100 encompasses a determination of whether an ongoing cardiac rhythm of the ECG of the patient is a shockable cardiac rhythm or a non-shockable cardiac rhythm. If the ongoing cardiac rhythm of ECG of the patient is determined to be a shockable cardiac rhythm during stage S116, then a stage S118 of flowchart 100 encompasses a delivery of a third shock to the patient and a stage S120 of flowchart 100 encompasses an administration of a third CPR protocol to the patient.

In one embodiment of stage S118, a biphasic shock energy of at least 120-200 joules can be delivered to the patient, or alternatively, a maximum available biphasic shock energy. In a second embodiment of stage S118, a monophasic shock energy of 360 joules can be delivered to the patient.

In one embodiment of stage S120, the responder is guided to administer a quality CPR to as set forth in the second CPR protocol the patient for a set time period (e.g., two (2) minutes). Additionally, an Amiodarone IV/IO dose of 3000 mg bolus can be delivered to the patient, and reversible causes can be treated (e.g., hypovolemia, hypoxia, hydrogen ion (acidosis), hypo-/hypercaloric, hypothermia, tension pneumothorax, cardiac tamponade, toxins, pulmonary thrombosis and coronary thrombosis).

Still referring to FIG. 3, if the ongoing cardiac rhythm of ECG of the patient is determined to be a non-shockable cardiac rhythm during an initial execution or iteration of stage S110 or stage S116, then a stage S130 of flowchart 100 encompasses a determination of a return of spontaneous circulation (ROSC) of the patient or no signs of ROSC of the patient. In one embodiment of stage S130, a determination of ROSC can be derived from pulse and blood pressure of the patient, an abrupt sustained increase in a maximum partial pressure of CO₂ at the end a breath should be ≥ 40 mm Hg, and/or a spontaneous arterial wave with intra-arterial monitoring.

If a ROSC of the patient is determined during S130, then a stage S132 of flowchart 100 encompasses a post-cardiac arrest care of the patient as known in the art of the present disclosure.

If a ROSC of the patient is not determined during S130, then a stage S122 of flowchart 100 encompasses an administration of the second CPR protocol of stage S114.

Still referring to FIG. 3, subsequent to an expiration of the set time period of stage S 122, a stage S 124 of flowchart 100 encompasses a determination of whether an ongoing cardiac rhythm of the ECG of the patient is a shockable cardiac rhythm or a non-shockable cardiac rhythm. If the ongoing cardiac rhythm of ECG of the patient is determined to be a shockable cardiac rhythm during stage S 124, then flowchart 100 proceeds to stage S 112 or S 118 for shock delivery as previously described. If the ongoing cardiac rhythm of ECG of the patient is determined to be a non-shockable cardiac rhythm during stage S 124, then a stage S 126 of flowchart 100 encompasses an administration of a fourth CPR protocol directed to treating reversible causes as previously described.

Still referring to FIG. 3, subsequent to an expiration of the set time period of stage S 126, a stage S 128 of flowchart 100 encompasses a determination of whether an ongoing cardiac rhythm of the ECG of the patient is a shockable cardiac rhythm or a non-shockable cardiac rhythm. If the ongoing cardiac rhythm of ECG of the patient is determined to be a shockable cardiac rhythm during stage S 128, then flowchart 100 proceeds to stage S 112 or S 118 for shock delivery as previously described. If the ongoing cardiac rhythm of ECG of the patient is determined to be a non-shockable cardiac rhythm during stage S 124, then flowchart 100 proceeds to stage S 130 for ROSC determination or non-existence as previously described.

Still referring to FIG. 3, subsequent to the CPR prep stage of S 102, if the cardiac rhythm of the ECG of the patient is determined to be a non-shockable cardiac rhythm during stage S 104, then flowchart 100 proceeds to stage S 122 as previously described.

In general, prior to the defibrillator accessories of the present disclosure, this protocol incorporates drug delivery, shock delivery and electrocardiogram (ECG) analysis whereby typically the responder will stop cardiopulmonary resuscitation (CPR) chest compressions to interpret the ECG for various purposes. The responder must also remember/manually record the rhythms of the patient, drugs provided, effect of drugs and which stages of the protocol have been performed by the responder (e.g., how many shocks were delivered).

The defibrillator accessories of the present disclosure provide for a more optimal execution of the protocol.

For example, in accordance with the previous description of FIG. 1, wearable biosensor 40 will facilitate an avoidance of excessive ventilation and an achievement of a targeted compression: ventilation ratio during CPR.

By further example, in accordance with the previous description of FIG. 1, wearable injector 50 will facilitate a determination of when to administer a drug/medication and upon such determination how much of such drug/medication should be administrated. This determination can be based on a history of medications (e.g., what medication has been provided and dose at what time).

By further example, in accordance with the previous description of FIG. 1, digital stethoscope 60 will facilitate a more accurate determination of ROSC.

By further example, in accordance with the previous description of FIG. 1, microphone 70 and light sensor 80 facilitate a sound and lighting of defibrillator 20 responsive to the ambient environment to thereby minimize any problems due to the ambient environment.

The present disclosure further provides additional features for a more optimal execution of the protocol of FIG. 3.

One such feature of the present disclosure is a language detection/selection method. Specifically, a defibrillator will typically be preset to a default language and provides prompts/instructions to a user independent of what language the user can understand. This can and has led to a user using a defibrator incorrectly. While some defibrators may have an option to change language, such option is not always clear, especially if the instructions are not in the user's language. Further for defibrators that can allow a user to change language prompts/instruction, such may need to be done manually/physically (e.g., pressing buttons) to get the defibrillator to switch languages. Even if/when successful in ultimately changing to a desired language, valuable time has been wasted that could negatively affect the outcome of the treatment. Moreover, known defibrillators today do not change/adjust the instructions based on the user's local culture.

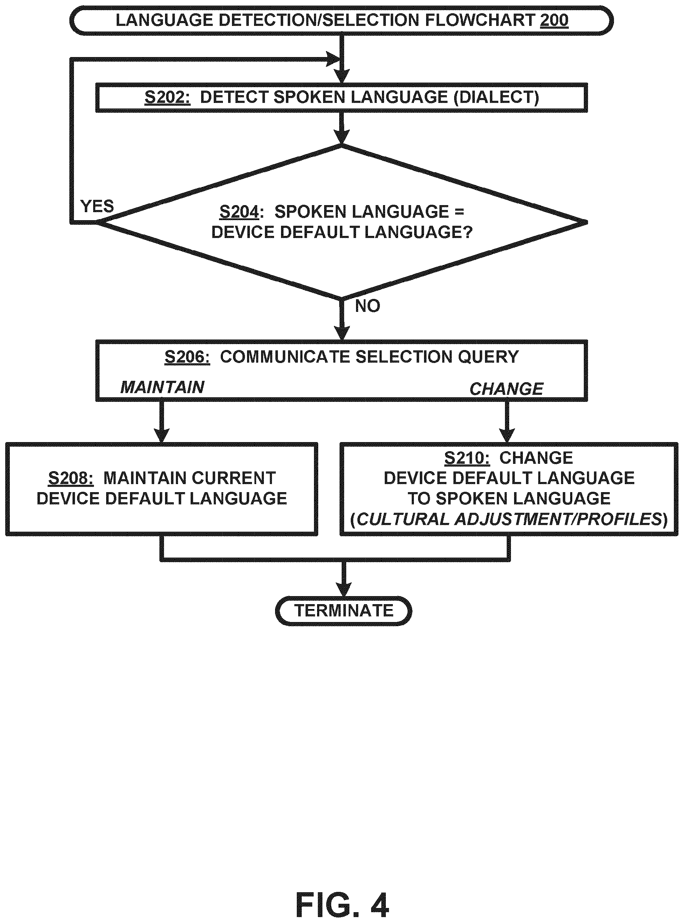

FIG. 4 illustrates a flowchart 200 representative of an exemplary embodiment of the language selection/detection method of the present disclosure. FIG. 4 will now be described in the context of defibrillator 20 (FIG. 1) executing flowchart 200 as a subroutine of resuscitation protocol manager 28a (FIG. 2). For purposes of the describing and claiming the present disclosure, the term "language" broadly encompasses a particular language with or without a particular dialect. For example, the term "language" as described and claimed can encompass English without a dialect or English with a Southern dialect.

Referring to FIG. 4, a stage S202 of flowchart 200 encompasses defibrillator 20 utilizing microphone 70 to detect a language being spoken by a user of and/or any person within the acoustic range of microphone 70, and a stage S204 determine if the detected spoken language is the same as the language designated by defibrillator 20 for audio outputs (including, e.g., text/written and/or verbal instructions and process flow). If, during stage S204 of flowchart 200, defibrillator 20 determines that the detected spoken language is different than the device default language for audio outputs, then the defibrillator 20 proceeds to a stage S206 of flowchart 200 to, via speaker 71 (e.g., a smart speaker) of defibrillator 20 to communicate a selection query to the user, in the detected spoken language and/or the device default language, if the user would like for the defibrator 20 to provide audio outputs, such as instructions, in the detected spoken language or to maintain the device default language. In practice, the selection query can be a selection of (a) an affirmative or a negative to maintaining the device default language, (b) an affirmative or negative to change the device default language to the detected spoken language or (c) a selection between maintaining the device default language and changing the device default language to the detected spoken language.

Upon a user selection of maintaining the device default language (e.g., audio selection, interface selection), defibrillator 20 proceeds to a stage S208 of flowchart 200 to execute the protocol (FIG. 3) therewith in accordance with device default language. Conversely, upon a user selection of detected spoken language (e.g., audio selection, interface selection), defibrillator 20 proceeds to a stage S210 of flowchart 200 to execute the protocol (FIG. 3) therewith in accordance with detected spoken language.

Further in practice, in addition to providing for switching to the detected spoken language, defibrillator 20 can automatically adjust the instructions based on the culture norms associated with the detected spoken language. For example, if the culture identified by defibrillator 20 based on the detected spoken language is prone to never removing a person's clothing in public, the adjusted instructions can include additional prompting and support to the user to remove the clothing to expose the chest for placing the electrodes/pad, emphasizing that it is acceptable to do so in this emergency situation to help save the patient's life. These adjusted instructions can be organized, for example, in preconfigured profiles corresponding to different languages and dialects of such languages.

Further, the profiles can use Artificial Intelligence (AI) so that the defibrillator 20 can guide users during an emergency based on the profile corresponding to different languages and dialects of such languages. For example, voice guidance can simulate an Emergency Medical Technician (or a doctor, authority figure, etc.), and their tone of voice, depending on the profile and what AI helps to determine which simulated persona (e.g., voice) would be most effective in providing real-time, personalized advice to the user during the emergency. Two-way AI communication, e.g., where a lay user can ask questions and AI responds with an answer based on the profile and in the device default language and/or the detected language and dialect if selected. In addition to guiding the user through the medical and treatment (e.g., defibrillation) protocol, this aspect of the profile will enable the defibrillator 20 to, e.g., instill confidence and help to keep the rescuer/user calm during a chaotic event, including unprompted prompts by the defibrillator 20 of encouragement, based on spoken feedback and/or physiological monitoring real-time feedback.

Examples can include:
- Responder: "He is turning blue, is that normal?"
   AI: "Yes, keep doing CPR!"
- Responder: "He started breathing"
   AI: "That's great, you can stop CPR, but leave the pads in place"
- AI: "You are doing great, keep it up! "
- AI: "I hear the sirens; help should be here soon."

An additional feature of the present disclosure is an agonal breathing therapy. Specifically, one of the deterrents to identifying sudden cardiac arrest is the patient's breathing. Labored or agonal breathing occurs in about half of all witnessed cardiac arrests and is associated with a higher chance of survival. However, labored, agonal and irregular breathing can be mistaken as breathing, and therefore calling 911, beginning CPR and deploying a defibrillator is not initiated.

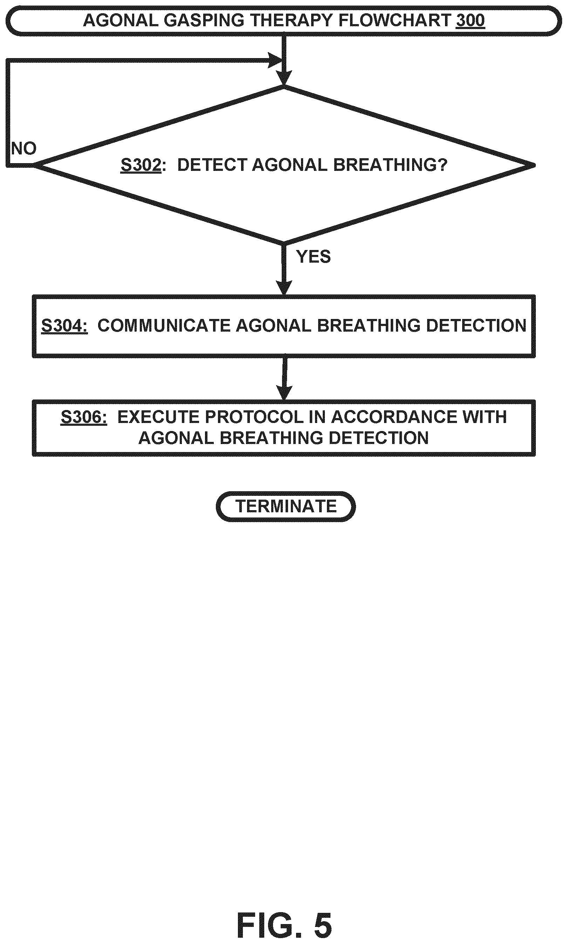

FIG. 5 illustrates a flowchart 300 representative of an exemplary embodiment of an agonal breathing therapy method of the present disclosure.

Referring to FIG. 5, a stage S302 of flowchart 300 encompasses defibrillator 20 determining if a patient is experiencing agonal breathing. In practice of stage S302, defibrillator 20 can incorporate technology as known in the art of the present disclosure for determining if a patient is experiencing agonal breathing via microphone 70 or can incorporate a messaging manager for receiving messages that the patient is experiencing agonal breathing. Stage S302 will typically occur when the defibrillator is not being used during rescue protocol of FIG. 3.

If defibrillator 20 determines a patient is experiencing agonal breathing, then a stage S304 of flowchart 300 encompasses defibrillator 20 to communicate the agonal breathing to a responder, which broadly encompasses an amateur operator of defibrillator 20, a professional technician of defibrillator 20, or an emergency center.

For example, defibrillator 20 can dial 911 or tell a smart speaker or smart phone to dial 911 and deploy emergency medical services whereby EMS can provide CPR and defibrillation upon arriving at the scene. Additionally, defibrillator 20 can sound an alarm (like a fire alarm) to alert anyone else in the residence whereby that person can deploy the AED knowing that emergency services is already on its way.

By further example, defibrillator 20 can communicate via speaker with a local responder to call 911 and deploy defibrillator 20 whereby a dispatcher can be able to direct the local responder as needed.

Still referring to FIG. 5, a stage S306 of flowchart 300 encompasses defibrillator 20 executing protocol (FIG. 3) in accordance with the agonal breathing. For example, based on a time period of detecting the agonal breathing, defibrillator 20 can determine if chest compressions or a defibrillating shock delivery should be initially administered to the patient as agonal breathing is indicative of the patient being in the early stages of a cardiac arrest. In the early stages ventilation and blood flow from CPR will be of less priority than an immediate defibrillation shock.

Also by example, the detection of agonal breathing by the defibrillator that is being used by a lay responder could be used to indicate that the arrest is early and the patient has not been down long (agonal breathing stops after a few minutes). For example, knowing an unwitnessed arrest is early is good information for the advanced caregivers, i.e., if this information was provided to the second tier responders/paramedics, it could help them with Termination of Resuscitation decisions and length of time spent trying to revive a patient in accordance with the protocol. For example, if the patient has agonal breathing indicating early stages of arrest, the responders will spend more time trying to revive the patient. This information could be conveyed by the AED itself to the advanced responders or 911 dispatchers could be notified so they can inform EMS.

From the description of FIGS. 1-5 herein, those having ordinary skill in the art will appreciate the numerous benefits of the present disclosure including, but not limited to, an incorporation of additional accessories to a defibrillator to enhance an execution of a resuscitation protocol by the defibrillator.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

One having ordinary skill in the art of defibrillators in view of the present disclosure shall appreciate that the present disclosure can apply to all defibrillators and related devices and systems, including, for example, Automated External Defibrillators (AEDs), professional devices such as Pre-hospital Defibrillators/Monitors, and In-hospital Defibrillators/Monitors.

Further, as one having ordinary skill in the art shall appreciate in view of the teachings provided herein, features, elements, components, etc. disclosed and described in the present disclosure/specification and/or depicted in the appended Figures and/or recited in the claims can be implemented in various combinations of hardware and software, and provide functions which may be combined in a single element or multiple elements. For example, the functions of the various features, elements, components, etc. shown/illustrated/depicted in the Figures and/or recited in the claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A defibrillation system for implementing a resuscitation protocol including a cardiopulmonary resuscitation protocol (CPR) and a shock protocol, the defibrillation system comprising:
at least one of:
a wearable biosensor (40) configured to monitor a ventilation of the patient;
a wearable injector (50) configured to administer a medication to the patient;
a digital stethoscope (60) configured to monitor a heart of the patient; and
at least one ambient sensor (70, 80) configured to monitor at least one of ambient sound and ambient light; and
a defibrillator (20) including a defibrillation controller (21) configured to implement the resuscitation protocol in accordance with one of:
a monitoring of the ventilation of the patient by the wearable biosensor (40);
an administering of the medication to the patient by the wearable injector (50);
a monitoring of the heart of the patient by the digital stethoscope (60); and
a monitoring of one of ambient sound and ambient light by the at least one ambient sensor (70, 80).

2. The defibrillation system of claim 1,
wherein the wearable biosensor (40) is further configured to generate a ventilation data indicative of the monitoring of the ventilation of the patient; and
wherein the defibrillation controller (21) is further configured to:
derive one of a ventilation rate and a ventilation volume from the ventilation data; and
execute the CPR protocol in accordance with the one of the ventilation rate and the ventilation volume.

3. The defibrillation system of claim 1,
wherein the wearable injector (50) is further configured to generate medication data indicative of one a timing of an administration of the medication to the patient and a quantity of the medication administrated to the patient; and
wherein the defibrillation controller (21) is further configured to execute at least one of the CPR protocol and the shock protocol in accordance with the shock data.

4. The defibrillation system of claim 1,
wherein the digital injector (50) is further configured to generate cardiac data indicative of the monitoring of the heart of the patient; and
wherein the defibrillation controller (21) is further configured to execute a return of spontaneous circulation determination shock based, at least in part, on the cardiac data.

5. The defibrillation system of claim 1,
wherein the at least one ambient sensor (70, 80) is further configured to generate ambient data indicative of at least one of the ambient sound and the ambient light; and
wherein the at least one of:
the defibrillation controller (21) is further configured to control at least one of an audible communication and a visual communication by the defibrillator (20) to a responder in accordance with the ambient data;
the defibrillation controller (21) is further configured to ascertain if the patient is experiencing agonal gasping based on the ambient data, and when the defibrillation controller (21) ascertains the patient is experiencing agonal gasping, control an agonal breathing detection communication by the defibrillator (20) to the responder and implement the resuscitation protocol based on the agonal gasping; and
the defibrillation controller (21) is further configured to communicate a selection query to the responder if the ambient data is indicating a detected spoken language of the responder is different than a device default language of the defibrillator (20), and to change the device default language of the defibrillator (20) to the spoken language of the responder upon receiving a selection communication from the responder of the detected spoken language.

6. A defibrillation controller (21) for controlling an implementation by a defibrillator (20) of a resuscitation protocol including a cardiopulmonary resuscitation protocol (CPR) and a shock protocol, the defibrillation controller (21) comprising:
a non-transitory machine-readable storage medium encoded with instructions for execution by at least one processor, the non-transitory machine-readable storage medium including the instructions to:
establish communication with at least one of:
a wearable biosensor (40) configured to monitor a ventilation of the patient;
a wearable injector (50) configured to administer a medication to the patient;
a digital stethoscope (60) configured to monitor a heart of the patient; and
at least one ambient sensor (70, 80) configured to monitor at least one of ambient sound and ambient light; and
control the implementation of the resuscitation protocol by the defibrillator (20) in accordance with one of a monitoring of the ventilation of the patient by the wearable biosensor (40), an administering of the medication to the patient by the wearable injector (50), a monitoring of the heart of the patient by the digital stethoscope (60), and a monitoring of one of ambient sound and ambient light by the at least one ambient sensor (70, 80).

7. The defibrillation controller (21) of claim 6,
wherein the wearable biosensor (40) is further configured to generate a ventilation data indicative of the monitoring of the ventilation of the patient; and
wherein the non-transitory machine-readable storage medium further includes instructions to:
derive one of a ventilation rate and a ventilation volume from the ventilation data; and
execute the CPR protocol in accordance with the one of the ventilation rate and the ventilation volume.

8. The defibrillation controller (21) of claim 6,
wherein the wearable injector (50) is further configured to generate medication data indicative of one a timing of an administration of the medication to the patient and a quantity of the medication administrated to the patient; and
the non-transitory machine-readable storage medium further includes instructions to execute at least one of the CPR protocol and the shock protocol in accordance with the shock data.

9. The defibrillation controller (21) of claim 6,
wherein the digital injector (50) is further configured to generate cardiac data indicative of the monitoring of the heart of the patient; and
the non-transitory machine-readable storage medium further includes instructions to execute a return of spontaneous circulation determination shock based, at least in part, on the cardiac data.

10. The defibrillation controller (21) of claim 6,
wherein the at least one ambient sensor (70, 80) is further configured to generate ambient data indicative of at least one of the ambient sound the ambient light; and
the non-transitory machine-readable storage medium further includes instructions to at least one of:
control at least one of an audible communication and a visual communication by the defibrillator (20) to a responder in accordance with the ambient data;
ascertain if the patient is experiencing agonal gasping based on the ambient data, and when the defibrillation controller (21) ascertains the patient is experiencing agonal gasping, controlling an agonal breathing detection communication by the defibrillator (20) to the responder and implementing the resuscitation protocol based on the agonal gasping; and
communicating a selection query to the responder if the ambient data is indicating a detected spoken language of the responder is different than a device default language of the defibrillator (20), and changing the device default language of the defibrillator (20) to the spoken language of the responder upon receiving a selection communication from the responder of the detected spoken language.

11. A defibrillation method executable by a defibrillator (20) for implementing a resuscitation protocol including a cardiopulmonary resuscitation protocol (CPR) and a shock protocol, the defibrillation method comprising:
at least one of:
monitoring, by a wearable biosensor (40), a ventilation of the patient;
administering, by a wearable injector (50), a medication to the patient;
monitoring, by a digital stethoscope (60) configured, a heart of the patient; and
monitoring, by at least one ambient sensor (70, 80), at least one of ambient sound and ambient light; and
implementing, by the defibrillator (20), the resuscitation protocol in accordance with one of:
the monitoring of the ventilation of the patient by the wearable biosensor (40);
the administering of the medication to the patient by the wearable injector (50);
the monitoring of the heart of the patient by the digital stethoscope (60); and
the monitoring of one of ambient sound and ambient light by the at least one ambient sensor (70, 80).

12. The defibrillation method of claim 11,
wherein the monitoring, by the wearable biosensor (40), the ventilation of the patient includes the wearable biosensor (40) generating a ventilation data indicative of the monitoring of the ventilation of the patient; and
wherein the implementing, by the defibrillator (20), of the resuscitation protocol includes:
the defibrillator (20) deriving one of a ventilation rate and a ventilation volume from the ventilation data; and
the defibrillator (20) executing the CPR protocol in accordance with the one of the ventilation rate and the ventilation volume.

13. The defibrillation method of claim 11,
wherein the administering, by the wearable injector (50), to the patient includes the wearable injector (50) generating medication data indicative of one a timing of an administration of the medication to the patient and a quantity of the medication administrated to the patient; and
wherein the implementing, by the defibrillator (20), of the resuscitation protocol includes the defibrillator (20) executing at least one of the CPR protocol and the shock protocol in accordance with the shock data.

14. The defibrillation method of claim 11,
wherein the monitoring, by the digital injector (50), of the heart of the patient includes the digital stethoscope (60) generating cardiac data indicative of the monitoring of the heart of the patient; and
wherein the implementing, by the defibrillator (20), of the resuscitation protocol includes the defibrillator (20) executing a return of spontaneous circulation determination shock based, at least in part, on the cardiac data.

15. The defibrillation method of claim 11,
wherein the monitoring, by the at least one ambient sensor (70, 80), of the ambient sound and ambient lights, includes the least one ambient sensor (70, 80) generating ambient data indicative of at least one of the ambient sound and the ambient light; and
wherein the implementing, by the defibrillator (20), of the resuscitation protocol includes at least one of:
the defibrillation controller (21) controlling at least one of an audible communication and a visual communication by the defibrillator (20) to a responder in accordance with the ambient data;
the defibrillation controller (21) ascertaining if the patient is experiencing agonal gasping based on the ambient data, and when the defibrillation controller (21) ascertains the patient is experiencing agonal gasping, controlling an agonal breathing detection communication by the defibrillator (20) to the responder and implementing the resuscitation protocol based on the agonal gasping; and
the defibrillation controller (21) communicating a selection query to the responder if the ambient data is indicating a detected spoken language of the responder is different than a device default language of the defibrillator (20), and changing the device default language of the defibrillator (20) to the spoken language of the responder upon receiving a selection communication from the responder of the detected spoken language.
